**Europäisches Patentamt**

**European Patent Office**

**Office européen des brevets**

(19)

(11) Publication number: **0 079 139**
**B1**

(12) # EUROPEAN PATENT SPECIFICATION

(45) Date of publication of patent specification: **07.01.88**

(51) Int. Cl.⁴: **C 12 Q 1/68, C 12 Q 1/04**

(21) Application number: **82305489.5**

(22) Date of filing: **15.10.82**

(54) **A method and reagent combination for the identification of microorganisms and the use of sandwich hybridization of nucleic acids therefor.**

(30) Priority: **16.10.81 FI 813251**

(43) Date of publication of application:
**18.05.83 Bulletin 83/20**

(45) Publication of the grant of the patent:
**07.01.88 Bulletin 88/01**

(84) Designated Contracting States:
**AT BE CH DE FR GB IT LI LU NL SE**

(56) References cited:
**GB-A-2 019 408**
**SU-A- 649 751**
**US-A-3 930 956**
**US-A-4 486 539**
**US-A-4 563 419**

**Molec. gen. Genet. (1980), vol. 179, pages 13-20**
**J. Inf. Diseases, vol. 142, no. 6 ( 980), pages 892-897**

**The file contains technical information submitted after the application was filed and not included in this specification**

(73) Proprietor: **Orion Corporation, Ltd.**
**Nilsiänkatu 10-14**
**SF-00510 Helsinki 51 (FI)**

(72) Inventor: **Ranki, Tuula Marjut**
**Keltasirkuntie 2**
**FI-02660 Espoó 66 (FI)**
Inventor: **Söderlund, Hans Erik**
**Salonkitie 19**
**FI-02940 Espoo 94 (FI)**

(74) Representative: **Hale, Stephen Geoffrey et al**
**J.Y. & G.W. Johnson Furnival House 14/18 High Holborn**
**London WC1V 6DE (GB)**

(56) References cited:
**J. Clin. Microbiol. (1980), vol. 12, no. 2, pages 226-234**

**Virology (1983), vol. 126, no. 32, pages 32-50**
**Proc. Natl. Acad. Sci. USA (1980), vol. 77, no. 11, pages 6851-6855**

**Meth. Enzym. vol. 65 (part I), 1980, pages 468-478**

Courier Press, Leamington Spa, England.

(56) References cited:
**Proc. Natl. Acad. Sci. USA (1980), vol.77, no. 11, pages 6376-6380**

**Proc. Natl. Acad. Sci. USA (1979), vol. 76,no. 9, pages 4360-4364**

**A.R. DUNN AND J.A. HASSELL, "Cell", vol. 12, no. 1, September 1977, MASSACHUSETTS INSTITUT OF TECHNOLOGY, pages 23-36**

## Description

The present invention relates to the use of sandwich hybridization of nucleic acids for the identification of microorganisms, and to a method and a combination of reagents for this purpose.

In traditional microorganism detection and identification techniques the presence of a microorganism in a given sample is demonstrated by isolation of the microbe in question. After enrichment cultivation the microorganism is identified either on the basis of its biochemical properties or with the use of immunological methods. These techniques require the microorganism in the sample to be viable. Identification by isolation is moreover a laborious method, which in the case of viruses may require 4—6 weeks.

The purpose of this invention is to provide a method in which the presence of a microorganism in a sample is demonstrated by the identification of its genetic material, the nucleic acid, with the aid of the sensitive and specific nucleic acid hybridization technique. In itself, nucleic acid hybridization is a known method for investigating the identity of nucleic acids. Complementary nucleic acid strands have the ability to form a tight double-stranded structure according to the rules of base pairing, and the resulting hybrid can be separated from the residual single-stranded nucleic acid.

Some methods based on the identification of nucleic acid(s) have already been applied to microorganism identification. Enterotoxigenic *Escherichia coli* have been identified from faecal samples by colony hybridization using the gene for toxin production as a probe. Positive hybridization is demonstrated by autoradiography (Moseley, S. L. et al., J. Infect. Dis (1980) *142*, 892—898). Colony hybridization is based on the method originally developed by Grünstein and Hogness (Proc. Natl. Acad. Sci. USA (1975) *72*, 3961—3965). Hybridization has also been used as a method to distinguish between Herpes simplex virus type 1 and type 2 (Brautigam, A. R. et al. J. Clin. Microbiol, (1980) *12*, 226—234), however not in rapid diagnostics but in typing of the virus after enrichment cultivation. In this method the double-stranded hybrid is separated by affinity chromatography from the fraction of nucleic acids remaining single-stranded in the solution.

It was recently published that DNA from cells infected with Epstein-Barr virus (the sample) had been fixed onto the filters after appropriate pretreatment. This nucleic acid was identified by hybridizing the filters with the radioactive probe and positive hybridization was detected by autoradiography (Brandsma, I. and Miller, K. (1980) Proc. Natl. Acad. Sci. USA *77*, 6851—6855).

The invention described herein is based on the sandwich hybridization technique (Dunn, A. R. and Hassell, J. A. (1977) Cell *12*, 23—36), which simplifies the handling of the sample and the detection of the hybrid. For this reason the technique is particularly suitable for diagnostic use.

Accordingly, the invention provides for the use of the technique of sandwich hybridization of nucleic acids for the identification of a microorganism or group of microorganisms present in a sample, said technique comprising contacting single-stranded nucleic acid from a microorganism or group of microorganisms to be identified with a pair of different nucleic acid reagents, both reagents of the pair being single-stranded and complementary with the microorganism-derived nucleic acid but essentially incapable of hybridizing directly with one another and one of the pair being nucleic acid fragment attached to a solid carrier whilst the other is a nucleic acid fragment labelled with a marker, whereby a labelled hybrid is formed attached to the solid carrier, the pair of nucleic acid reagents being selected according to the microorganism or group of microorganisms expected to be present in the sample and the correctness of the identification being tested by detection of the extent of formation of a labelled hybrid attached to the solid carrier.

The invention also provides a reagent combination for use in the identification of single-stranded nucleic acid sequences from microorganisms by means of sandwich hybridization, characterised in that it comprises a plurality of different pairs of nucleic acid reagents, each pair being composed of a first nucleic acid reagent which is a single-stranded nucleic acid fragment attached to a solid carrier and a second nucleic acid reagent which is a single-stranded nucleic acid fragment labelled with a marker, both nucleic acid reagents in each pair being complementary with a particular nucleic acid sequence which might require to be identified and being capable of interacting with said nucleic acid sequence in a sample to form a labelled hybrid attached to the solid carrier but being essentially incapable of hybridizing directly with one another, and the reagents of different pairs being complementary with different nucleic acid sequences which might require to be identified.

Furthermore, the invention provides a kit for the detection of single-stranded nucleic acid sequences from microorganisms by a one-step sandwich hybridization test, the kit comprising in packaged combination in a container at least one pair of nucleic acid reagents, the or each pair of reagents being composed of

(a) a first nucleic acid reagent comprising a single-stranded fragment of nucleic acid affixed to a solid carrier and capable of forming a double-stranded hybrid molecule by base-pairing with a portion of a nucleic acid sequence which might require to be identified using the kit, and

(b) a second nucleic acid reagent comprising a single-stranded fragment of nucleic acid labelled with a detectable label and capable of forming a double-stranded hybrid molecule by complementary base pairing with a different portion of the same nucleic acid sequence as base-pairs with the first nucleic acid

**0 079 139**

reagent of the same pair, but such that the second nucleic acid reagent does not hybridize directly with the first nucleic acid reagent of any pair.

Additionally, the invention provides a method of identifying microorganisms or groups of microorganisms present in a sample, by means of sandwich hybridization of nucleic acids, using a pair of different nucleic acid reagents both complementary to and capable of hybridizing with nucleic acid from the same known microorganism or group of microorganisms, one of the pair of nucleic acid reagents being a single-stranded nucleic acid fragment attached to a solid carrier and the other being a single-stranded nucleic acid fragment labelled with a marker, whereby on successful hybridization a labelled hybrid attached to the solid carrier is formed, the method being characterised in that a plurality of microorganisms or groups of microorganisms present in a single undivided sample are identified by bringing nucleic acid from the microorganisms in the sample, after they have been rendered single-stranded, into contact with a plurality of the pairs of completely different nucleic acid reagents, reagents in different pairs being complementary with and capable of hybridizing with nucleic acid from different known microorganisms or groups of microorganisms, and detecting the formation or non-formation of labelled hybrids attached to a solid carrier in respect of each of the pairs of nucleic acid reagents.

Moreover, the invention provides a method of checking the identity of a single-stranded nucleic acid from a microorganism by means of sandwich hybridization of the nucleic acid sequence with a first nucleic acid reagent which is a single-stranded nucleic acid fragment attached to a solid carrier and a second nucleic acid reagent which is a single-stranded nucleic acid fragment labelled with a marker, both nucleic acid reagents being complementary with a nucleic acid sequence to be identified and interacting with said nucleic acid sequence, if it has the expected identity, to form a labelled hybrid attached to the solid carrier, characterised in that the sandwich hybridization is performed as a one-step sandwich hybridization, the single-stranded nucleic acid sequence being contacted in a single step with the first and second nucleic acid reagents, which have been selected according to the expected identity of the nucleic acid sequence and so that they do not significantly hybridize directly with one another, and the correctness of the identification of the nucleic acid sequence being tested by detection of the formation of the labelled hybrid attached to the solid carrier.

By means of this invention all the microorganisms or groups of microorganisms present can be identified from one and the same sample, which contains denaturated single-stranded nucleic acid strands from the microorganisms, without sample division. A pair of the different nucleic acid reagents is required for each microorganism or group of microorganisms to be identified. The pair of reagents are two separate nucleic acid fragments derived from the genome of the microorganism to be identified, which have no sequences in common but preferably are situated close to each other in the genome. The reagents can be prepared directly from the microbial genomes or by using the established recombinant DNA techniques. Of the two nucleic acid fragments, one is fixed to a solid carrier, preferably a nitrocellulose filter, after being denaturated and the other, also in single-stranded form, is labelled with a suitable marker label. When these nucleic acid reagents, two different reagents for each microorganism or group of microorganisms to be identified, are placed in contact with the single-stranded nucleic acids to be identified in the sample, the nucleic acids in the sample couple to the complementary nucleic acid fragments on the solid carrier. The hybrids thus formed on the carrier become labelled by coupling to the labelled complementary nucleic acid fragments. The labelled nucleic acid fragments do not hybridize directly with the nucleic acid fragments attached to the solid carrier, but hybridize only with the correct single stranded nucleic acids originating from the sample. Thus only those carriers to which the complementary nucleic acids from the sample have hybridized can become labelled. These carriers are easily washed and the presence of the label measured by established methods.

The invention can in principle be used for the identification of all organisms containing either DNA or RNA, such as viruses, bacteria, fungi and yeasts. It has the specific advantage of permitting identification of all the bacteria and viruses possibly in question at the same time and from the same sample, regardless of whether the microorganisms contain DNA or RNA. By suitable combination of pairs of reagents it is possible to develop "kits" such that each microorganism to be identified has its own specific pair of reagents, one attached to a solid carrier and the other labelled. All the solid carriers included in the reagent combinations can be added to the sample simultaneously, along with the labelled nucleic acid reagents. When hybridization has taken place, the solid carriers are washed and their labelling is measured. The only carrier or carriers to become labelled are those which contain sequences complementary to the microbial genome in the investigated sample.

The invention can be used, for example, in medical microbiology, veterinary microbiology, food hygiene investigations and microbiological investigation of plant diseases. Suitable sample materials are for example all animal- and plant tissue homogenates, and human and animal secretions such as blood, faeces and nasal- and urethral mucus. The procedure can be made sufficiently sensitive to detect the microorganism levels normally present in clinical samples. Preliminary enrichment of the microorganism(s) present in the sample by cultivation is of course possible before the identification test and in some cases would be essential. The invention is also suitable for the investigation of samples from which the microorganism can no longer be cultivated but which contain considerable amounts of microbial debris (e.g. after the commencement of antibiotic treatment), or when cultivation of the microorganism is

4

particularly laborious and difficult (e.g. anaerobic bacteria, which are present in large numbers in suppurative samples in the case of infections caused by anaerobes).

The invention may be utilized, for example, to provide reagent combinations for detection and identification of the following groups of pathogenic microorganisms by appropriate formation of the necessary pairs of nucleic acid reagents. Of course reagent combinations need not contain pairs of reagents specific to each one of the organisms or groups of organisms named under the various individual headings.

Respiratory infections:

a) Bacteria: β-haemolytic streptococci (A-group), *Haemophilus influenzae,* Pneumococci, *Mycoplasma pneumoniae,* mycobacteria

b) Viruses: Influenza A, Influenza B, Parainfluenza 1—3 Respiratory syncytial virus, adenoviruses, corona viruses, rhinoviruses

Diarrhoeas:

a) Bacteria: salmonellae, shigellae, *Yersinia enterocolitica*, enterotoxigenic *E. coli, Clostridium difficile,* campylobacteria

b) Viruses: rotaviruses, parvoviruses, adenoviruses, enteroviruses

Venereal diseases:

a) Bacteria: *Neisseria gonorrhoeae, Treponema pallidum, Chlamydia* trachomatis

b) Viruses: Herpes simplex-virus

c) Yeasts: *Candida albicans*

d) Protozoa: *Trichomonas vaginalis*

Sepsis:

a) Bacteria: β-haemolytic streptococci (A-group), pneumococci, enterobacteria as a single group

Food hygiene:

a) Bacteria: salmonella and *Clostridium perfringens*

Depending on the choice of reagents the specificity of the test can be limited to a single microorganism or a small group of microorganisms (e.g. salmonella bacteria) or to a wider group (e.g. enterobacteriaceae) by choosing identifying reagents from the area of a common gene.

The nucleic acid reagents required in the sandwich hybridization technique described in this invention may be produced by recombinant DNA technology or directly from the genome.

By way of illustration the reagent production and test procedure for Example 1 will now be described, but it will be appreciated by those skilled in the art that appropriate modifications may be made without departing from the basic principles of the invention, and that these methods and procedures can be adapted for the production of other pairs of nucleic acid reagents.

Reagents

Adenovirus type 2 (strain deposited at KTL, i.e. the Public Health laboratory, Helsinki) was cultivated and purified and DNA was isolated (Petterson, U. and Sambrook, J. (1973) J. Mol. Biol. *73*, 125—130) (referred to hereafter as $Ad_2$-DNA). The DNA was digested with BamHI-restriction enzyme (BRL, i.e. Bethesda Research Laboratories), which cuts the DNA into four reproducible fragments. Of these four fragments two were inserted into the BamHI-site of the vector plasmid pBR322 (BRL) with the aid of T4-ligase (BRL). (The fragments were not separated before ligation, but the insert added to the plasmid was in each case identified only after cloning). Subsequently the bacterial host (*E. coli* HB101 (K12) gal⁻, pro⁻, leu⁻, hrs⁻, hrm⁻, recA, str$^r$, F⁻) (obtained from KTL) was transformed with the plasmid DNA also containing recombinant plasmids, i.e. molecules which had accepted fragments of the adenovirus-DNA (Cohen, S. N. et al. (1972) Proc. Natl. Acad. Sci. USA *69*, 2110—2114). Among the transformed bacterial clones those that most probably contained the recombinant plasmid were chosen. Ampicillin-resistance and tetracycline-resistance are transferred to the bacterium by the pBR322-plasmid (Bolivar F. et al. (1977) Gene *2*, 95—113). Bacteria containing the recombinant plasmid are, however, sensitive to tetracycline, because the BamHI-restriction site is within the tetracycline gene and the insertion of foreign DNA into this region destroys the gene. The insertion of the plasmid was characterized after plasmid enrichment by determining the size of the restriction fragments after BamHI digestion using agarose gel electrophoresis. The adjacent BamHI D- and C-fragments of the $Ad_2$-DNA (cf. gene map) were chosen as reagents (Söderlund, H. et al. (1976) Cell *7*, 585—593). The desired recombinant plasmids, $Ad_2$C-pBR322, KTL No. E231 and $Ad_2$D-pBR322, KTL No. EH230, were cultivated and purified as has been described in the literature (Clewell, D. B. and Helinski, D. R. (1969) Proc. Natl. Acad. Sci. USA *62*, 1159—1166).

The recombinant plasmid $Ad_2$D-pBR322 was used as the carrier-bound reagent. It is not necessary for the present invention to remove the plasmid sequences, because the sample does not contain pBR322-sequences. However, for radioactive labelling the insert nucleic acid was separated from pBR322-DNA after BamHI-digestion with the aid of agarose gel electrophoresis. The C-fragment was isolated from LGT-agarose (Marine Colloids, Inc.) by phenol extraction or electro-elution (Wieslander, L. (1979) Anal. Biochem. *98*, 305—309) and concentrated by ethanol precipitation.

It is particularly desirable to subclone the nucleic acid fragment chosen for labelling in a separate vector, in order to avoid the hybridization background resulting from the direct hybridization with the carrier-bound reagent of the residual plasmid sequences, contaminating the labelled nucleic acid reagent. The single-stranded DNA-phage M13 mp7 (BRL), to which DNA fragments obtained by BamHI digestion can easily be transferred, may be used as an optimal vector (Messing, J. et al. (1981) Nucleic Acids Res. *9*, 309—323).

Attachment of DNA to the solid carrier (filter)

The recombinant plasmid Ad$_2$D-pBR322 was denatured to a single stranded form and nicked randomly at several sites by treatment with 0.2 N NaOH (5 min. 100°C), whereafter the DNA was chilled and, immediately prior to transference to the filter, neutralized and pipetted to the transfer solution, 4×SSC medium on ice (SSC=0.15 M NaCl, 0.015 M Na-citrate). The filters (Schleicher and Schüll BA85 nitrocellulose) were thoroughly wetted in 4×SSC solution (about 2 h) before the application of DNA. The DNA was attached to the filter in a dilute solution (0.5—1.0 µg/ml) by sucking the solution through the filter in a weak vacuum. The filter is capable of absorbing DNA up to about 180 µg/cm$^2$ (Kafatos, F. C. et al. (1979) Nucleic Acids Res. *7*, 1541—1552). We have used DNA-concentrations of between 0.5 µg DNA/2.5 cm diameter of filter and 1.0 µg DNA/0.7 cm diameter of filter.

After DNA-filtration the filters are washed in 4×SSC, dried at room temperature and finally baked in a vacuum oven at 80°C for 2 h, after which the DNA on the filters remains stable and the filters can be stored for long periods at room temperature (Southern, E. M. (1975) J. Mol. Biol. *98*, 503—517).

Labelling of the radioactive nucleic acid fragment

The radioactive label used was the $^{125}$I-isotope. This isotope can be detected using γ-counters, which are available in most large laboratory units. The half-life of the isotope is 60 days, for which reason the utilization period of $^{125}$I-labelled reagents is about 4 months.

"Nick-translation" labelling

The principle of this method is to displace one of the nucleotides in the nucleic acid with a radioactive one, upon which the whole DNA molecule becomes labelled. This is carried out according to the method published by Rigby, P. W. J. et al. (J. Mol. Biol. (1977) *113*, 237—251). In the reaction the DNA becomes labelled when the solution contains $^{125}$I-labelled deoxynucleoside triphosphate as substrate, in this case $^{125}$I-dCTP (Radiochemical Centre, Amersham: >1500 Ci/mmol). Under optimal conditions a specific activity of 10$^9$ cpm/µg DNA can be obtained. The labelled DNA is purified from nucleotides remaining in the reaction mixture by simple gel filtration, e.g. using BioGel® P30 (BioRad).

Other labelling methods

The single-stranded nucleic acid reagent produced in M13 mp7-phage is labelled by chemical iodination, in which the reactive $^{125}$I is bound covalently to the nucleic acid (Commerford, S. L. (1971) Biochemistry *10*, 1993—2000, Orosz, J. M. and Wetmur, J. G. (1974) Biochemistry *13*, 5467—5473). Alternatively, the nucleic acid can be made radioactive by end-labelling with radioactive nucleotides by means of terminal transferase (Roychoudhury, R. and Wu, R. (1980) Meth. Enzymol. *65*, 43—62).

The reagent preparation described above relates to microbes of which the genetic material is in the form of DNA. In the case of RNA viruses the cloning of genome fragments can take place after a DNA copy (cDNA) of the virus RNA has been made with the aid of reverse transcriptase, followed by DNA-polymerase to copy the second DNA strand, thereafter the DNA may be cloned as described above (Salser, W. (1979) in Genetic Engineering, Ed. A. M. Chakrabarty, CRC Press, pp. 53—81).

The most suitable cloning method should be chosen depending on the microbe used. The hosts as well as the vectors can vary. Possibilities include the λ-phage as vector, other plasmids, cosmids, and cloning, e.g. in *Bacillus subtilis* bacteria. (Recombinant DNA, Benchmarck Papers in Microbiology, Vol. 15, Eds. K. J. Denniston and L. W. Enqvist, Dowden, Hutchinson and Ross, Inc. (1981); Ish-Horowicz, D. and Burke, J. F. (1981) Nucleic Acids Res. *9*, 2989—2998).

Performance of the test
Sample treatment

The microbial nucleic acid to be investigated must be released from within the microbe itself and also from the infected cells, after which it must be denatured to the single-stranded form. Virus genomes can be liberated by treating the sample material with 1% sodium dodecylsulphate (SDS) and destroying the proteins protecting the genome by proteinase K-treatment (1 mg/ml, 37°C, 60 min). Bacterial samples must in addition be broken down using lysozyme- and EDTA-treatment.

If the sample contains large quantities of viscous high-molecular weight cellular-DNA, this must be sheared at a few sites in order to reduce its viscosity, e.g. by sonication or by passing the sample a few times through a fine needle.

## Hybridization

Hybridization takes place e.g. in 50% formamide (deionized, stored at −20°C), in 4×SSC, Denhardt solution (Denhardt, D. T. (1966) Biochem. Biophys. Res. Commun. 23, 641—646) containing 1% SDS and 0.5 mg/ml DNA (salmon sperm or calf thymus) at 37°C and usually overnight for 16—20 hours. The filters chosen for the test are incubated in a suitable vessel, to which the hybridization mixture is added and the hybridization is started. The hybridization mixture contains (a) the pretreated sample to which is added the radioactive nucleic acid reagent(s), which are denatured together by boiling for 5 minutes followed by quick cooling at 0°C; (b) concentrated formamide-, SSC- and Denhardt-solutions, which are pipetted to the denatured and cooled nucleic acid mixture (a). After mixing, the hybridization mixture is pipetted to the filters in the hybridization vessel. After hybridization the filters are carefully washed and counted individually in the γ-counter.

The invention will now be further described, by way of illustration only, with the aid of some practical Examples.

## Example 1
Detection of adenovirus by the sandwich hybridization method (Table 1)

The details of the test are clarified in the text to Table 1. The sandwich hybridization method can detect virus-DNA from a solution, but the viral genome can equally well be detected from infected cells.

The hybridization background is measured in a tube containing only the filter and the labelled nucleic acid reagent, without the sample. The background results from the pBR322 sequences occurring in the labelled nucleic acid reagent. These sequences hybridize directly with the filter without the sample mediating it. The filters containing calf thymus and no DNA are used in the test as controls, indicating on the one hand the specificity of hybridization and on the other the level of the nonspecific background arising e.g. from insufficient washing.

In the following tables the background due to the reagents has been subtracted from the cpm-values hybridized to the filters.

TABLE 1
Adenovirus test

| Sample | Adeno [1] | Filters (cpm) Calf thymus [2] | Blank [3] |
|---|---|---|---|
| Adenovirus type 2-DNA (BRL) (500 ng) | 9000 | 49 | — |
| HeLa-cells ($6 \times 10^5$) infected with adenovirus | 8200 | — | — |

Filters:
[1] $Ad_2D$-pBR322-plasmid, 2 µg
[2] Calf thymus DNA 1 µg (Boehringer Mannheim)
[3] Blank (no DNA).

Labelled nucleic acid reagent:
$Ad_2$-BamHI C-fragment, purified specific activity $90 \times 10^6$ cpm/µg (200000 cpm $^{125}$I/reaction).

Hybridization:
50% formamide, 4×SSC
Denhardt solution, containing 0.5 mg/ml salmon sperm DNA and 1% SDS, 37°C, 16 h.

Washing:
0,1×SSC, room temperature, 40 min.

Samples:
Adenovirus type 2 DNA (BRL)
Infection with type 2 adenovirus took place in HeLa-cells. The cells were then disrupted by treatment with 1% SDS, followed by digestion with 1 mg/ml proteinase-K-enzyme (Sigma) for 30 min 37°C. Before denaturation the sample was passed through a fine needle. The values appearing in the table have been corrected by subtraction of the reagent background, obtained by carrying out a similar hybridization but without sample.

## Example 2
Detection of an RNA-virus with the aid of sandwich hybridization (Table 2)

The model RNA-virus used was the Semliki Forest virus (prototype strain, obtained from the London School of Hygiene and Tropical Medicine), of which the genome is single-stranded RNA. Using the virus genome as a template cDNA was produced, which was cloned into the PstI site of pBR322 plasmid as described by Garoff et al. (Proc. Natl. Acad. Sci. (1980) USA 77 6376—6380). The recombinant plasmid thus obtained is pKTH312 KTL No. EH 232. The insert of this plasmid originating from the virus genome is about 1400 nucleotides long and is from the structural protein area, approximately from nucleotide 200 to nucleotide 1600 when numbering is started from the beginning of the structural genes (Garoff, H. et al. 1980). For the production of reagent the whole recombinant plasmid pKTH312 was linearized with EcoPI restriction enzyme (BRL). The sequence originating from the Semliki Forest virus does not contain recognition sites for the EcoRI-enzyme, and the linearized plasmid was cut into two fragments using XhoI-enzyme (BRL). The restriction site of the latter was located within the Semliki Forest virus sequence. The larger EcoRI-XhoI-fragment A (about 3900 base pairs) was attached to the filter and the smaller fragment B (about 1850 base pairs) was labelled with $^{125}$I using the nick translation technique.

Both free Semliki Forest virus and virus-infected cells were used as samples in this test. In both cases the virus-specific nucleic acids of the sample were composed entirely of RNA.

TABLE 2

Detection of Semliki Forest virus with the aid of the sandwich hybridization method

| | Filters (cpm) | | |
| --- | --- | --- | --- |
| Sample | Semliki Forest virus [1] | Calf thymus [2] | Blank [3] |
| Semliki Forest virus 30 μg | 3340 | — | 33 |
| Cells infected with Semliki Forest virus (5×10⁵) | 2698 | 8 | 10 |
| Non-infected cells | 10 | 5 | 8 |

Filters:
   [1] EcoRI-XhoI-fragment A (1.2 μg) of the pKTH312 plasmid
   [2] Calf thymus DNA 1 μg
   [3] Blank (no DNA).

Labelled nucleic acid reagents:
   EcoRI-XhoI-fragment B of the plasmid pKTH312, specific activity 90×10⁶ cpm/μg (DNA (200000 cpm $^{125}$I/reaction).

Hybridization:
   As described in Table 1.

Washing:
   As described in Table 1.

Samples:
   Semliki Forest virus (30 μg) was disrupted with SDS before the test. The infected cells were handled as described in Table 1. The infection with Semliki Forest virus was carried out in BHK-21 cells.
   The values given in the table have been corrected for reagent background, obtained from a similar hybridization without sample.

Example 3
A virus sample in which the viral messenger RNA is detected with the aid of the sandwich hybridization method (Table 3)
   The sandwich hybridization reagents were produced from SV40-virus DNA (BRL) by cutting the DNA into two parts using PstI-enzyme (Boehringer Mannheim) as described by Lebowitz and Weissman (Curr. Topics in Microbiol. Immunol. 87, 43—172) and the fragments were isolated and purified by agarose gel electrophoresis. Fragment A (4000 base pairs) was radioactively labelled with $^{125}$I by nick translation and fragments B (1220 base pairs) was attached to the filter.
   The DNA fragments were chosen so that each contained areas coding for both early and late messengers. Thus fragment B contains about 700 bases from the structural protein gene VP1 and over 600 bases from the gene for early messengers. Because the DNA of SV40 virus is in itself a covalently closed ring, it cannot be detected by the test before linearization. Therefore, when infected cells are used as the

8

**0 079 139**

sample it is possible to test how well the method is adaptable to the detection of RNA copies of the viral genome. As can be seen from the results in Table 3, the test is excellently suited to the investigation of infected cells. The table also demonstrates that the same reagents can be used to investigate both the viral DNA and mRNA made from it.

TABLE 3
Detection of SV40-virus by the sandwich hybridization technique

| Sample | Filters (cpm) | | |
|---|---|---|---|
| | SV40 [1] | Calf thymus [2] | Blank [3] |
| Test 1<br>SV40 viral DNA (50 ng)<br>(linearized) | 20061 | 159 | 104 |
| No sample | — | — | — |
| Test 2<br>CV1-cells infected with<br>SV40-virus 40 h after<br>infection ($10^6$ cells) | 30814 | 294 | 580 |
| Non-infected cells | — | — | — |

Filters:
[1] The shorter fragment Pstl B (0.2 µg) of the circular SV40-virus DNA digested with Pstl-restriction enzyme
[2] Calf thymus DNA 1 µg
[3] Blank (no DNA).

Labelled nucleic acid reagent:
The longer Pstl A-fragment of the SV40-virus DNA, specific activity $28 \times 10^6$ cpm/µg DNA (200000 cpm $^{125}$I/reaction).

Hybridization:
As described in Table 1.
The hybridization time is 40 h.

Washing:
As described in Table 1.

Samples:
SV40-virus DNA (BRL) was linearized with EcoRI restriction enzyme (BRL). CV1-cells (Biomedical Centre, Upsala University) were infected with SV40-virus (obtained from Janice Y. Chou and Robert G. Martini, NIH, Bethesda) and the cells were harvested 40 h after infection. Treatment of the sample was as described in Table 1.
The values presented in the table have been corrected for reagent background, obtained from a similar hybridization carried out without sample.

Example 4
Detection of Bacillus amyloliquefaciens by sandwich hybridization (Table 4)
The reagents were fragments of the α-amylase gene of *B. amyloliquefaciens* E18 (Technical Research Centre of Finland, VTT), which were isolated for the purpose of this test from the recombinant plasmid pKTH10 (Palva, I., et al. (1981) Gene, *15* 43—51) by treatment with restriction enzyme and subsequent agarose gel electrophoresis. The fragments used for this test were the Clal-EcoRI fragment (460 base pairs) (Clal Boehringer Mannheim) and the EcoRI-BamHI fragment (1500 base pairs). The EcoRI-BamHI fragment was attached to the filter and the Clal-EcoRI fragment was labelled with $^{125}$I by nick translation.
As can be seen from Table 4 the *B. amyloliquefaciens* in a sample was identifiable by sandwich hybridization on the basis of the single α-amylase gene. *E. coli* gave a negative result in this test (indistinguishable from the background).

9

# 0 079 139

### TABLE 4
### Bacterial diagnostics by sandwich hybridization

| Sample | Filters (cmp) | | |
|---|---|---|---|
| | α-amylase [1] | Calf thymus [2] | Blank [3] |
| pKTH10-plasmid-DNA (linearized) 1 μg | 5773 | 47 | — |
| No sample | — | — | — |
| E. coli HB101 (10⁹) | — | — | — |
| Bacillus amylolique-faciens (3×10⁹) | 3377 | — | — |
| Bacillus amylolique-faciens (10⁹) | 2871 | — | — |

Filters:
[1] The EcoRI-BamHI fragment of the α-amylase gene from plasmid pKTH10, 0.35 μg
[2] Culf thymus DNA, 1 μg
[3] Blank (no DNA).

Labelled nucleic acid reagent:
The ClaI-EcoRI fragment of the α-amylase gene from plasmid pKTH10, specific activity $35 \times 10^6$ cpm/μg (200000 cpm ¹²⁵I/reaction.

Hybridization:
As described in Table 1.

Washing:
As described in Table 1.

Samples:
Bacterial samples were treated with lysozyme (67 μg/ml) for 30 min at 37°C; 5 mM EDTA was added to E. coli samples, too. After the treatment SDS was added to all the samples (final concentration 2%), which were then passed twice through a fine needle to reduce their viscosity before being denatured by boiling as described in the text relating to handling of samples.
The values appearing in the table have been corrected for reagent background, obtained from a similar hybridization without sample.

Example 5
An example of a reagent combination kit based on the sandwich hybridization method (Table 5)
The samples investigated in this test were cells infected by three viruses (adenovirus, SV40 virus and Herpex simplex virus) and a sample containing Bacillus amyloliquefaciens bacteria. The following reagents were all simultaneously added to each sample, 5 filters, each containing one type of DNA from SV40 virus, adenovirus, Bacillus amyloliquefaciens α-amylase gene and calf thymus, as well as a filter containing no DNA at all; in addition 200000 cpm of each of the following labelled nucleic acid reagents: SV40 virus-, adenovirus- and α-amylase gene DNA-reagent.
Our example shows that it is possible, without division or dilution of the sample, to investigate simultaneously a suitable series of microbes by adding the reagent combination to the sample. The sample may contain both viral and bacterial nucleic acid. The filters can be recognized by a sign (=mark, tags), which identifies the sequence it contains and tags, which microbe was attached/hybridized to it. The signs can be numbers or letters, e.g. 1 or SV40 2 or Ad etc. or other markers as * for SV40 or Δ for AD or o for Bacillus.

10

TABLE 5
A kit based on the filter hybridization technique

| Sample | Filters (cpm) | | | | |
|---|---|---|---|---|---|
| | SV40 [1] | Adeno [2] | α-amylase [3] | Calf thymus [4] | Blank [5] |
| Cells infected with SV40 virus ($10^6$) | 18390 | 2 | 13 | 22 | 31 |
| Cells infected with adenovirus type 2 ($6 \times 10^5$) | — | 8750 | 5 | 13 | — |
| Cells infected with Herpex simplex virus ($10^6$) | — | — | — | 5 | 13 |
| *Bacillus amylolique-faciens* ($10^9$) | 15 | 8 | 6500 | 16 | 5 |
| Non-infected cells | — | — | — | — | — |

Filters:
[1] As in Table 3
[2] As in Table 1
[3] As in Table 4
[4] Calf thymus DNA, 1 μg
[5] Blank (no DNA).

Labelled nucleic acid reagents:
SV40 virus as in Table 3
Adenovirus as in Table 1
α-amylase gene as in Table 4.

Hybridization:
As in Table 1.

Washing:
As in Table 1.

Samples:
Cell samples infected with SV40 virus and adenovirus have been described in Tables 3 and 1, respectively. $10^6$ Vero cells were infected with Herpes simplex virus type 1. The cells were harvested 20 h post infection as cytopathic effect could be observed. The sample was treated as described for adenovirus infected cells (Table 1).

*Bacillus amyloliquefaciens* sample:
As in Table 4.
The values in the table are corrected for reagent background, obtained by carrying out a similar hybridization without sample.

Example 6
Detection of *Escherichia coli* by sandwich hybridization (Table 6)
The reagents were prepared from the ompA-gene (outer membrane protein A-gene) of *Escherichia coli*.
The hybrid plasmids pKTH40 and pKTH45, used as starting material, were prepared from the pTU100 plasmid described by Henning et al. (1979) Proc. Natl. Acad. Sci. USA *76*, 4360—4364.
The plasmid pKTH45 (deposited at KTL or National Public Health Institute Helsinki No. EH 257), used as a filter reagent, was composed of 740 base pairs from 5'-terminal end of the ompA-gene inserted into the pBR322-plasmid.
The plasmid pKTH40 contains 300 base pairs from the 3'-terminal end of the ompA-gene and the immediately following 1400 base pairs from the genome of *E. coli*. The pKTH40 plasmid was cleaved with

the BamHI restriction enzyme to receive the DNA fragment of *E. coli*, which contains the 1700 base pairs mentioned above. This fragment was transferred to the single-stranded bacteriophage M13mp7 according to the methods described by (Messing et al. (1981), Nucl. Acids Res. *9*, 309—321, Heidecker et al. (1980), Gene *10*, 69—73, Gardner et al. (1981), Nucl. Acids Res. *9*, 2871—2888). The recombinant-phage mKTH1207 (deposited at KTL No. EH 256) was labelled with $^{125}$I-isotope as described on page 6 under the heading "Other labelling methods" and was used as a probe in the sandwich hybridization method.

DNA from disrupted *E. coli* cells, as well as isolated, purified DNA from *E. coli* can be detected by sandwich hybridization as shown in Table 6.

TABLE 6

Detection of *Escherichia coli* by sandwich hybridization

| Sample | Filters (cpm) | | |
|---|---|---|---|
| | ompA [1] | Calf thymus [2] | Blank [3] |
| *E. coli* K12 HB101 DNA a) $2\times10^7$ | · 282 | — | — |
| *E. coli* K12 HB101 DNA a) $2\times10^8$ | 2206 | — | — |
| *E. coli* K12 HB101 Cells b) $2\times10^7$ | 1113 | — | — |
| *E. coli* K12 HB101 Cells b) $2\times10^8$ | 2327 | 12 | 5 |

a) number of DNA-molecules, b) number of cells.

Filters:
 [1] pKTH45 plasmid 1,088 µg ($2\times10^{11}$ molecules)
 [2] Calf thymus DNA 1,088 µg
 [3] Blank (no DNA).

Labelled nucleic acid reagents:
 mKTH1207, specific activity $8\times10^7$ cpm/µg DNA (200000 cpm/reaction).

Hybridization:
 4×SSC, 1×Denhardt solution without BSA (bovine serum albumin), 0,25% SDS, 200 µg/ml Herring sperm DNA, 17,5 h, +65.

Washing:
 As described in Table 1.

Samples:
 *E. coli* K12 HB101-DNA was isolated according to the Marmur-method described by Marmur (1961) J. Mol. Biol. *3*, 208—218. DNA was denatured at 7 mM NaOH, 100°C, 5 min.
 The cells were treated with lysozyme (500 µg/ml), EDTA (70 mM +37°C, 30 min), SDS (0,25%, +65°C) and the free DNA was denatured by boiling at 14 mM NaOH, +100°C, 5 min).
 The values presented in the table have been corrected for reagent background obtained from a similar hybridization without sample.

**Claims**

1. A reagent combination for use in the identification of single-stranded nucleic acid sequences from microorganisms by means of sandwich hybridization, characterised in that it comprises a plurality of different pairs of nucleic acid reagents, each pair being composed of a first nucleic acid reagent which is a single-stranded nucleic acid fragment attached to a solid carrier and a second nucleic acid reagent which is a single-stranded nucleic acid fragment labelled with a marker, both nucleic acid reagents in each pair being complementary with a particular nucleic acid sequence which might require to be identified and being capable of interacting with said nucleic acid sequence in a sample to form a labelled hybrid attached to the solid carrier but being essentially incapable of hybridizing directly with one another, and the reagents of different pairs being complementary with different nucleic acid sequences which might require to be identified.

2. A reagent combination as claimed in claim 1, characterised in that each first nucleic acid reagent is a nucleic acid fragment in the form of a recombinant plasmid attached to a solid carrier known for use as a carrier for recombinant plasmids.

3. A reagent combination as claimed in claim 1 or 2, characterised in that the solid carrier for one or more of the first nucleic acid reagents is nitrocellulose.

4. A reagent combination as claimed in any of claims 1 to 3, characterised in that each second nucleic acid reagent is a nucleic acid fragment in the form of a recombinant plasmid labelled with a marker known for use in the labelling of recombinant plasmids.

5. A reagent combination as claimed in any of claims 1 to 4, characterised in that one or more of the second nucleic acid reagents is radioactively labelled.

6. A reagent combination according to any of claims 1 to 5, characterised in that the two nucleic acid reagents of the same pair are in each case fragments produced directly from the genome of the microorganism to which they are complementary and subsequently rendered single-stranded.

7. A reagent combination according to any of claims 1 to 5, characterised in that the two nucleic acid reagents of the same pair are in each case fragments produced by means of a recombinant DNA technique and subsequently rendered single-stranded.

8. A reagent combination according to any of claims 1 to 7, characterised in that one or more of the pairs of nucleic acid reagents is complementary to and capable of hybridizing with a single-stranded nucleic acid sequence from a bacterium or virus associated with a respiratory infection or diarrhoea, a bacterium, virus, yeast or protozoon associated with a venereal disease or a bacterium associated with sepsis or with failures in food hygiene.

9. A reagent combination according to any of claims 1 to 8, characterised in that one of the pairs of nucleic acid reagents is complementary to and capable of hybridizing with a single-stranded nucleic acid sequence from adenovirus.

10. A reagent combination according to claim 9, characterised in that said pair of nucleic acid reagents consists of the adenovirus recombinant plasmid Ad$_2$D-pBR322, which consists in the Ad$_2$-BamHI D-fragment inserted into the BamHI-site of pBR322, attached to a solid carrier and the adenovirus Ad$_2$-BamHI C-fragment labelled with a marker.

11. A reagent combination according to any of claims 1 to 10, characterised in that one of the pairs of nucleic acid reagents is complementary to and capable of hybridizing with a single-stranded nucleic acid sequence from an RNA virus.

12. A reagent combination according to any of claims 1 to 11, characterised in that one of the pairs of nucleic acid reagents is complementary to and capable of hybridizing with a single-stranded nucleic acid sequence from SV40 virus.

13. A reagent combination according to claim 12, characterised in that said pair of nucleic acid reagents consists of the PstI B fragment of SV40 virus attached to a solid carrier and the PstI A fragment of SV40 virus labelled with a marker.

14. A reagent combination according to any of claims 1 to 13, characterised in that one of the pairs of nucleic acid reagents is complementary to and capable of hybridizing with a single-stranded nucleic acid sequence from *Bacillus amyloliquefaciens*.

15. A reagent combination according to claim 14, characterised in that said pair of nucleic acid reagents consists of the EcoRI-BamHI fragment of the α-amylase gene of the *Bacillus amyloliquefaciens* plasmid pKTH10 attached to a solid carrier and the ClaI-EcoRI fragment of the α-amylase gene of the *Bacillus amyloliquefaciens* plasmid pKTH10 labelled with a marker.

16. A reagent combination according to any of claims 1 to 15, characterised in that one of the pairs of nucleic acid reagents is complementary to and capable of hybridizing with a single-stranded nucleic sequence acid from enterotoxigenic *E. coli*.

17. A kit for use in the detection of single-stranded nucleic acid sequences from microorganisms by a one-step sandwich hybridization test, the kit comprising in packaged combination in a container at least one pair of nucleic acid reagents, the or each pair of reagents being composed of

(a) a first nucleic acid reagent comprising a single-stranded fragment of nucleic acid affixed to a solid carrier and capable of forming a double-stranded hybrid molecule by base-pairing with a portion of a nucleic acid sequence which might require to be identified using the kit, and

(b) a second nucleic acid reagent comprising a single-stranded fragment of nucleic acid labelled with a detectable label and capable of forming a double-stranded hybrid molecule by complementary base pairing with a different portion of the same nucleic acid sequence as base-pairs with the first nucleic acid reagent of the same pair, but such that the second nucleic acid reagent does not hybridize directly with the first nucleic acid reagent of any pair.

18. A kit as claimed in claim 17, characterised in that the solid carrier is nitrocellulose.

19. A kit as claimed in claim 17 or 18, characterised in that the label is selected from radioisotopes.

20. The use of the technique of sandwich hybridization of nucleic acid for the identification of a microorganism or group of microorganisms present in a sample, said technique comprising contacting single-stranded nucleic acid from a microorganism or group of microorganisms to be identified with a pair

13

of different nucleic acid reagents, both reagents of the pair being single-stranded and complementary with the microorganism-derived nucleic acid but essentially incapable of hybridizing directly with one another and one of the pair being a nucleic acid fragment attached to a solid carrier whilst the other is a nucleic acid fragment labelled with a marker, whereby a labelled hybrid is formed attached to the solid carrier, the pair of nucleic acid reagents being selected according to the microorganism or group of microorganisms expected to be present in the sample and the correctness of the identification being tested by detection of the extent of formation of a labelled hybrid attached to the solid carrier.

21. The use of the technique of sandwich hybridization of nucleic acids according to claim 20, characterised in that a plurality of the pairs of nucleic acid reagents are used, each pair being complementary with nucleic acid derived from a different microorganism or group of microorganisms, any one of which is expected to be present in the sample.

22. The use of the technique of sandwich hybridization of nucleic acids according to claim 20, characterised in that a plurality of different microorganisms or groups of microorganisms are present in the sample and are detected in one and the same undivided sample by the use of a variety of different pairs of the nucleic acid reagents complementary with nucleic acids from the different microorganisms or groups of microorganisms expected to be present in the sample.

23. The use of the technique of sandwich hybridization of nucleic acids according to any of claims 20 to 22, characterised in that both reagents of the or each pair of nucleic acid reagents are added to the sample for a hybridization in a one-step procedure and when hybridization has taken place the extent of labelling of the solid carrier or carriers due to the formation of a labelled hybrid is measured.

24. A method of identifying microorganisms or groups of microorganisms present in a sample, by means of sandwich hybridization of nucleic acids, using a pair of different nucleic acid reagents both complementary to and capable of hybridizing with nucleic acid from the same known microorganism or group of microorganisms, one of the pair of nucleic acid reagents being a single-stranded nucleic acid fragment attached to a solid carrier and the other being a single-stranded nucleic acid fragment labelled with a marker, whereby on successful hybridization a labelled hybrid attached to the solid carrier is formed, the method being characterised in that plurality of a microorganisms or groups of microorganisms present in a single undivided sample are identified by bringing nucleic acids from the microorganisms in the sample, after they have been rendered single-stranded, into contact with a plurality of the pairs of completely different nucleic acid reagents, the reagents in different pairs being complementary with and capable of hybridizing with nucleic acid from different known microorganisms or groups of microorganisms, and detecting the formation or non-formation of labelled hybrids attached to a solid carrier in respect of each of the pairs of nucleic acid reagents.

25. A method as claimed in claim 24, characterised in that the two nucleic acid components of each pair are added simultaneously to the sample.

26. A method of checking the identity of a single-stranded nucleic acid from a microorganism by means of sandwich hybridization of the nucleic acid sequence with a first nucleic acid reagent which is a single-stranded nucleic acid fragment attached to a solid carrier and a second nucleic acid reagent which is a single-stranded nucleic acid fragment labelled with a marker, both nucleic acid reagents being complementary with a nucleic acid sequence to be identified and interacting with said nucleic acid sequence, if it has the expected identity, to form a labelled hybrid attached to the solid carrier, characterised in that the sandwich hybridization is performed as a one-step sandwich hybridization, the single-stranded nucleic acid sequence being contacted in a single step with the first and second nucleic acid reagents, which have been selected according to the expected identity of the nucleic acid sequence so that they do not significantly hybridize directly with one another, and the correctness of the identification of the nucleic acid sequence being tested by detection of the formation of the labelled hybrid attached to the solid carrier.

27. A method as claimed in claim 26, characterised in that a plurality of different pairs of first and second nucleic acid reagents is employed simultaneously in the one-step sandwich hybridization, each pair of reagents being selected according to a different expected identity of the nucleic acid sequence.

28. A method as claimed in claim 26, characterised in that a plurality of different single-stranded nucleic acid sequences is present and a plurality of different pairs of first and second nucleic acid reagents is employed simultaneously in the one-step sandwich hybridization, the different pairs of nucleic acid reagents being selected according to an expected identity of different ones of the nucleic acid sequences.

29. A method as claimed in any of claims 25 to 28, characterised in that the or each nucleic acid to be identified is inherently double-stranded and is denatured to the single-stranded form prior to the one-step sandwich hybridization, the denatured material being contacted with the pairs of nucleic acid reagents so that the formation of a labelled hybrid attached to the solid carrier competes with the reconstitution of the double-stranded nucleic acid sequence by reversal of the denaturing.

30. A method as claimed in any of claims 25 to 29, characterised in that the or each first nucleic acid reagent is a nucleic acid fragment in the form of or derived from a recombinant plasmid attached to a solid carrier known for use as a carrier for recombinant plasmids or for nucleic acid fragments from recombinant plasmids.

31. A method as claimed in any of claims 25 to 30, characterised in that the solid carrier for the first nucleic acid reagent or one or more of the first nucleic acid reagents is nitrocellulose.

# 0 079 139

32. A method as claimed in any of claims 25 to 31, characterised in that the or each second nucleic acid reagent is a nucleic acid fragment in the form of or derived from a recombinant plasmid labelled with a detectable marker known for use in the labelling of recombinant plasmids.

33. A method as claimed in any of claims 25 to 29 or 31, characterised in that the second nucleic acid reagent or one or more of the second nucleic acid reagents is radioactively labelled.

**Patentansprüche**

1. Reagenzkombination zur Verwendung bei der Identifizierung einsträngiger Nukleinsäuresequenzen aus Mikroorganismen durch Sandwich-Hybridisierung, dadurch gekennzeichnet, dass sie mehrere verschiedene Paare von Nukleinsäurereagenzien enthält, wobei jedes Paar aus einem ersten Nukleinsäurereagenz, das ein einsträngiges, an einen festen Träger gebundenes Nukleinsäurefragment darstellt, und einem zweiten Nukleinsäurereagenz besteht, das ein einsträngiges, mit einem Indikator markiertes Nukleinsäurefragment darstellt, wobei die beiden Nukleinsäurereagenzien in einem Paar jeweils mit einer bestimmten, gegebenenfalls zu identifizierenden Nukleinsäuresequenz komplementär und zur Wechselwirkung mit dieser Nukleinsäuresequenz in einer Probe unter Bildung eines markierten, an den festen Träger gebundenen Hybrids fähig sind, aber im wesentlichen nicht direkt miteinander hybridisieren können, und wobei die Reagenzien verschiedener Paare mit verschiedenen, gegebenenfalls zu identifizierenden Nukleinsäuresequenzen komplementär sind.

2. Reagenzkombination nach Anspruch 1, dadurch gekennzeichnet, dass das erste Nukleinsäurereagenz jeweils ein Nukleinsäurefragment in form eines an einen festen Träger gebundenen Rekombinationsplasmids ist, der zur Verwendung als Träger für Rekombinationsplasmide bekannt ist.

3. Reagenzkombination nach Anspruch 1 oder 2, dadurch gekennzeichnet, dass der feste Träger für ein oder mehrere der ersten Nukleinsäurereagenzien Nitrocellulose ist.

4. Reagenzkombination nach einem der Ansprüche 1 bis 3, dadurch gekennzeichnet, dass das zweite Nukleinsäurereagenz jeweils ein Nukleinsäurefragment in Form eines Rekombinationsplasmids ist, das mit einem zur Verwendung bei der Markierung von Rekombinationsplasmiden bekannten Indikator markiert ist.

5. Reagenzkombination nach einem der Ansprüche 1 bis 4, dadurch gekennzeichnet, dass ein oder mehrere der zweiten Nukleinsäurereagenzien radioaktiv markiert sind.

6. Reagenzkombination nach einem der Ansprüche 1 bis 5, dadurch gekennzeichnet, dass die beiden Nukleinsäurereagenzien im selben Paar jeweils direkt aus dem Genom des Mikroorganismus, mit dem sie komplementär sind, hergestellt und danach einsträngig gemacht werden.

7. Reagenzkombination nach einem der Ansprüche 1 bis 5, dadurch gekennzeichnet, dass die beiden Nukleinsäurereagenzien im selben Paar durch eine DNS-Rekombinationstechnik hergestellt und danach einsträngig gemacht werden.

8. Reagenzkombination nach einem der Ansprüche 1 bis 7, dadurch gekennzeichnet, dass eines oder mehrere der Paare Nukleinsäurereagenzien mit einer einsträngigen Nukleinsäuresequenz aus einem für eine Infektion der Atemwege oder Durchfall verantwortlichen Bakterium oder Virus, einem für eine Geschlechtskrankheit verantwortlichen Bakterium, Virus, einer Hefe oder einem Protozoon oder einem für Sepsis oder unzureichende Lebensmittelhygiene verantwortlichen Bakterium komplementär und damit hybridisierbar sind.

9. Reagenzkombination nach einem der Ansprüche 1 bis 8, dadurch gekennzeichnet, dass eines der Paare Nukleinsäurereagenzien mit einer einsträngigen Nukleinsäuresequenz aus Adenovirus komplementär und damit hybridisierbar ist.

10. Reagenzkombination nach Anspruch 9, dadurch gekennzeichnet, dass dieses Paar Nukleinsäurereagenzien aus dem an einen festen Träger gebundenen Adenovirus-Rekombinationsplasmid $Ad_2D$-pBR322, das aus dem an der BamHI-Stelle von pBR322 eingeschalteten $Ad_2$-BamHI-D-Fragment besteht, und dem mit einem Indikator markierten $Ad_2$-BamHI-C-Fragment aus Adenovirus besteht.

11. Reagenzkombination nach einem der Ansprüche 1 bis 10, dadurch gekennzeichnet, dass eines der Paare Nukleinsäurereagenzien mit einer einsträngigen Nukleinsäuresequenz aus einem RNS-Virus komplementär und damit hybridisierbar ist.

12. Reagenzkombination nach einem der Ansprüche 1 bis 11, dadurch gekennzeichnet, dass eines der Paare Nukleinsäurereagenzien mit einer einsträngigen Nukleinsäuresequenz aus SV40-Virus komplementär und damit hybridisierbar ist.

13. Reagenzkombination nach Anspruch 12, dadurch gekennzeichnet, dass dieses Paar Nukleinsäurereagenzien aus dem an einen festen Träger gebundenen PstI-B-Fragment aus SV40-Virus und dem mit einem Indikator markierten PstI-A-Fragment aus SV40-Virus besteht.

14. Reagenzkombination nach einem der Ansprüche 1 bis 13, dadurch gekennzeichnet, dass eines der Paare Nukleinsäurereagenzien mit einer einsträngigen Nukleinsäuresequenz aus Bacillus amyloliquefaciens komplementär und damit hybridisierbar ist.

15. Reagenzkombination nach Anspruch 14, dadurch gekennzeichnet, dass dieses Paar Nukleinsäurereagenzien aus dem EcoRI-BamHI-Fragment aus dem an einen festen Träger gebundenen α-Amylasegen des Plasmids pKTH10 aus Bacillus amyloliquefaciens und dem mit einem Indikator markierten ClaI-EcoRI-Fragment des α-Amylasegens des Plasmids pKTH10 aus Bacillus amyloliquefaciens besteht.

15

16. Reagenzkombination nach einem der Ansprüche 1 bis 15, dadurch gekennzeichnet, dass eines der Paare Nukleinsäurereagenzien mit einer einsträngigen Nukleinsäuresequenz aus enterotoxigenem E. coli komplementär und damit hybridisierbar ist.

17. Ausrüstung zur Verwendung beim Nachweis einsträngiger Nukleinsäuresequenzen aus Mikroorganismen durch einen einstufigen Sandwich-Hybridisierungstest, dadurch gekennzeichnet, dass sie in einem Behälter mindestens ein Paar Nukleinsäurereagenzien in einer Kombinationspackung enthält, wobei das bzw. jedes Paar Reagenzien such aus

(a) einem ersten Nukleinsäurereagenz, das aus einem einsträngigen, an einen festen Träger gebundenen und zur Bildung eines doppelsträngigen Hybridmoleküls durch Basenpaarung mit einem Teil einer gegebenenfalls unter Verwendung der Ausrüstung zu identifizierenden Nukleinsäuresequenz befähigten Nukleinsäurefragment besteht, und

(b) einem zweiten Nukleinsäurereagenz, das aus einem einsträngigen, mit einem nachweisbaren Indikator markierten und zur Bildung eines doppelsträngigen Hybridmoleküls durch komplementäre Basenpaarung mit einem unterschiedlichen Teil derselben Nukleinsäuresequenz wie die Basenpaare mit dem ersten Nukleinsäurereagenz im selben Paar befähigten Nukleinsäurefragment besteht, wobei aber das zweite Nukleinsäurereagenz nicht direkt mit dem ersten Nukleinsäurereagenz irgendeines Paares hybridisiert, zusammensetzt.

18. Ausrüstung nach Anspruch 17, dadurch gekennzeichnet, dass als fester Träger Nitrocellulose vorliegt.

19. Ausrüstung nach Anspruch 17 oder 18, dadurch gekennzeichnet, dass der Indikator unter Radioisotopen ausgewählt ist.

20. Anwendung der Sandwich-Hybridisierungstechnik für Nukleinsäure zur Identifizierung eines in einer Probe vorhandenen Mikroorganismus bzw. einer Mikroorganismengruppe, wobei diese Technik dadurch gekennzeichnet ist, dass man einsträngige Nukleinsäure aus einem zu identifizierenden Mikroorganismus bzw. einer Mikroorganismengruppe mit einem Paar verschiedener Nukleinsäurereagenzien in Berührung bringt, wobei beide Reagenzien des Paars einsträngig und mit der vom Mikroorganismus abgeleiteten Nukleinsäure komplementär, aber im wesentlichen miteinander nicht direkt hybridisierbar sind und ein Glied des Paares ein an einen festen Träger gebundenes Nukleinsäurefragment ist, während das andere ein mit einem Indikator markiertes Nukleinsäurefragment darstellt, wodurch sich ein an den festen Träger gebundenes markiertes Hybrid bildet, wobei das Paar Nukleinsäurereagenzien entsprechend dem in der Probe zur erwartenden Mikroorganismus bzw. der Mikroorganismengruppe ausgewählt und die Richtigkeit der Identifizierung durch Erfassung des Ausmasses der Bildung eines an den festen Träger gebundenen markierten Hybrids nachgewiesen wird.

21. Anwendung der Sandwich-Hybridisierungstechnik für Nukleinsäuren nach Anspruch 20, dadurch gekennzeichnet, dass man mehrere Paare Nukleinsäurereagenzien verwendet, wobei jedes Paar mit von einem verschiedenen Mikroorganismus bzw. einer Mikroorganismengruppe, wovon einer in der Probe zu erwarten ist, abgeleiteter Nukleinsäure komplementär ist.

22. Anwendung der Sandwich-Hybridisierungstechnik für Nukleinsäuren nach Anspruch 20, dadurch gekennzeichnet, dass mehrere verschiedene Mikroorganismen bzw. Mikroorganismengruppen in der Probe vorliegen und in derselben ungeteilten Probe durch Verwendung einer Reihe unterschiedlicher Paare Nukleinsäurereagenzien, die mit Nukleinsäuren aus den verschiedenen, in der Probe zu erwartenden Mikroorganismen bzw. Mikroorganismengruppen komplementär sind, nachgewiesen werden.

23. Anwendung der Sandwich-Hybridisierungstechnik für Nukleinsäuren nach einem der Ansprüche 20 bis 22, dadurch gekennzeichnet, dass beide Reagenzien aus dem bzw. jedem Paar Nukleinsäurereagenzien zur Hybridisierung der Probe in einer einstufigen Arbeitsweise zugesetzt werden und nach erfolgter Hybridisierung das Ausmass der Markierung des bzw. der festen Träger(s) durch Bildung eines markierten Hybrids gemessen wird.

24. Methode zur Identifizierung von in einer Probe vorliegenden Mikroorganismen bzw. Mikroorganismengruppen durch Sandwich-Hybridisierung von Nukleinsäuren unter Verwendung eines Paars verschiedener Nukleinsäurereagenzien, die beide mit Nukleinsäuren aus demselben bekannten Mikroorganismus bzw. Mikroorganismengruppen komplementär und damit hybridisierbar sind, wobei ein Glied des Paars Nukleinsäurereagenzien ein einsträngiges, an einen festen Träger gebundenes Nukleinsäurefragment und das andere ein einsträngiges, mit einem Indikator markiertes Nukleinsäurefragment ist, wodirch sich bei gelungener Hybridisierung ein an den festen Träger gebundenes markiertes Hybrid bildet, dadurch gekennzeichnet, dass mehrere in einer einzigen ungeteilten Probe vorhandene Mikroorganismen bzw. Mikroorganismengruppen identifiziert werden, indem man Nukleinsäuren aus den Mikroorganismen in der Probe, nachdem sie einsträngig gemacht wurden, mit mehreren der Paare völlig verschiedener Nukleinsäurereagenzien in Berührung bringt, wobei die Reagenzien in unterschiedlichen Paaren mit Nukleinsäure aus verschiedenen bekannten Mikroorganismen bzw. Mikroorganismengruppen komplementär und damit hybridisierbar sind, und die Bildung oder Nichtbildung an einen festen Träger gebundener markierter Hybride für jedes der Paare Nukleinsäurereagenzien nachweist.

25. Methode nach Anspruch 24, dadurch gekennzeichnet, dass die beiden Nukleinsäurekomponenten jedes Paares der Probe gleichzeitig zugesetzt werden.

26. Methode zum Nachprüfen der Identität einer einsträngigen Nukleinsäure aus einem Mikroorganismus durch Sandwich-Hybridisierung der Nukleinsäuresequenz mit einem ersten Nukleinsäure-

# 0 079 139

reagenz, das ein einsträngiges, an einen festen Träger gebundenes Nukleinsäurefragment darstellt, und einem zweiten Nukleinsäurereagenz, das ein einsträngiges, mit einem Indikator markiertes Nukleinsäurefragment darstellt, wobei die beiden Nukleinsäurereagenzien mit einer zu identifizierenden Nukleinsäuresequenz komplementär sind und damit in Wechselwirkung treten, wenn sie die erwartete Identität besitzt, zur Bildung eines an den festen Träger gebundenen, markierten Hybrids, dadurch gekennzeichnet, dass die Sandwich-Hybridisierung einstufig erfolgt, wobei die einsträngige Nukleinsäuresequenz in einer einzigen Stufe mit den ersten und zweiten Nukleinsäurereagenzien in Berührung gebracht wird, die entsprechend der erwarteten Identität der Nukleinsäuresequenz so ausgewählt wurden, dass sie im wesentlichen nicht direkt miteinander hybridisieren, und die Richtigkeit der Identifizierung der Nukleinsäuresequenz durch Nachweis der Bildung des an den festen Träger gebundenen, markierten Hybrids festgestellt wird.

27. Methode nach Anspruch 26 dadurch gekennzeichnet, dass mehrere verschiedene Paare erster und zweiter Nukleinsäurereagenzien gleichzeitig bei der einstufigen Sandwich-Hybridisierung eingesetzt werden, wobei die Reagenzienpaare jeweils entsprechend einer unterschiedlichen erwarteten Identität der Nukleinsäuresequenz ausgewählt werden.

28. Methode nach Anspruch 26, dadurch gekennzeichnet, dass mehrere verschiedene einsträngige Nukleinsäuresequenzen vorliegen und mehrere verschiedene Paare erste und zweite Nukleinsäurereagenzien gleichzeitig bei der einstufigen Sandwich-Hybridisierung eingesetzt werden, wobei die verschiedenen Paare Nukleinsäurereagenzien entsprechend der erwarteten Identität unterschiedlicher Nukleinsäuresequenzen ausgewählt werden.

29. Methode nach einem der Ansprüche 26 bis 28, dadurch gekennzeichnet, dass die bzw. jede zu identifizierende Nukleinsäure an sich doppelsträngig ist und vor der einstufigen Sandwich-Hybridisierung zur einsträngigen Form denaturiert wird, wobei man das denaturierte Material mit den Paaren Nukleinsäurereagenzien in Berührung bringt, so dass die Bildung eines an den festen Träger gebundenen, markierten Hybrids mit der Rückbildung der doppelsträngigen Nukleinsäuresequenz durch Umkehr der Denaturierung konkurriert.

30. Methode nach einem der Ansprüche 26 bis 29, dadurch gekennzeichnet, dass das bzw. jedes erste Nukleinsäurereagenz als Nukleinsäurefragment in Form eines an einen festen, zur Verwendung als Träger für Rekombinationsplasmide oder Nikleinsäurefragmente daraus bekannten Träger gebundenen Rekombinationsplasmids vorliegt oder davon abgeleitet ist.

31. Methode nach einem der Ansprüche 26 bis 30, dadurch gekennzeichnet, dass als fester Träger für das erste Nukleinsäurereagenz bzw. ein oder mehrere der ersten Nukleinsäurereagenzien Nitrocellulose vorliegt.

32. Methode nach einem der Ansprüche 26 bis 31, dadurch gekennzeichnet, dass das bzw. jedes zweite Nukleinsäurereagenz als Nukleinsäurefragment in Form eines mit einem zur Verwendung bei der Markierung von Rekombinationsplasmiden bekannten, nachweisbaren Indikator markierten Rekombinationsplasmids vorliegt oder davon abgeleitet ist.

33. Methode nach einem der Ansprüche 26 bis 29 oder 31, dadurch gekennzeichnet, dass das zweite Nukleinsäurereagenz bzw. eines oder mehrere der zweiten Nukleinsäurereagenzien radioaktiv markiert sind.

## Revendications

1. Une combinaison de réactifs pour l'utilisation dans l'identification de séquences d'acides nucléiques à simple brin provenant de microorganismes au moyen de l'hybridation en sandwich, caractérisée en ce qu'elle comprend une pluralité de différents couples de réactifs acides nucléiques, chaque couple étant composé d'un premier réactif acide nucléique qui est un fragment d'acide nucléique à simple brin lié à un support solide, et un second réactif acide nucléique qui est un fragment d'acide nucléique à simple brin, marqué à l'aide d'un marqueur, les deux réactifs acides nucléiques dans chaque couple étant complémentaires d'une séquence d'acide nucléique particulière qui peut avoir besoin d'être identifiée, et étant capables d'interagir avec ladite séquence d'acide nucléique dans un échantillon pour former un hybride marqué lié au support solide, mais étant sensiblement incapables de s'hybrider directement l'un avec l'autre, et les réactifs de différents couples étant complémentaires de différentes séquences d'acides nucléiques qui peuvent avoir besoin d'être identifiées.

2. Une combinaison de réactifs selon la revendication 1, caractérisée en ce que chaque premier réactif acide nucléique est un fragment d'acide nucléique sous forme d'un plasmide recombinant lié à un support solide connu pour son emploi en tant que support de plasmides recombinants.

3. Une combinaison de réactifs selon la revendication 1 ou 2, caractérisée en ce que le support solide pour un ou plusieurs des premiers réactifs acides nucléiques est la nitrocellulose.

4. Une combinaison de réactifs selon l'une quelconque des revendications 1 à 3, caractérisée en ce que chaque second réactif acide nucléique est un fragment d'acide nucléique sous forme d'un plasmide recombinant marqué à l'aide d'un marqueur, connu pour son emploi dans le marquage des plasmides recombinants.

5. Une combinaison de réactifs, selon l'une quelconque des revendications 1 à 4, caractérisée en ce qu'un ou plusieurs des seconds réactifs acides nucléiques est marqué de manière radioactive.

6. Une combinaison de réactifs selon l'une quelconque des revendications 1 à 5, caractérisée en ce que

17

**0 079 139**

les deux réactifs acides nucléiques du même couple sont dans chaque cas des fragments produits directement à partir du génome du microorganisme dont ils sont complémentaires, et rendus ultérieurement à simple brin.

7. Une combinaison de réactifs selon l'une quelconque des revendications 1 à 5, caractérisée en ce que les deux réactifs acides nucléiques du même couple sont dans chaque cas des fragments produits au moyen d'une technique à l'ADN recombinant, et rendus ultérieurement à simple brin.

8. Une combinaison de réactifs selon l'une quelconque des revendications 1 à 7, caractérisée en ce qu'un ou plusieurs des couples de réactifs acides nucléiques est complémentaire d'une séquence d'acide nucléique à simple brin et capable de s'hybrider avec ladite séquence d'acide nucléique à simple brin provenant d'une bactérie ou d'un virus associé à une infection respiratoire ou à une diarrhée, d'une bactérie, d'un virus, d'une levure ou d'un protozoaire associé à une maladie vénérienne, ou d'une bactérie associée à une sepsie ou à des manques d'hygiène alimentaire.

9. Une combinaison de réactifs selon l'une quelconque des revendications 1 à 8, caractérisée en ce que l'un des couples de réactifs acides nucléiques est complémentaire d'une séquence d'acide nucléique à simple brin et capable de s'hybrider avec ladite séquence provenant d'un adénovirus.

10. Une combinaison de réactifs selon la revendication 9, caractérisée en ce que ledit couple de réactifs acides nucléiques est constitué du plasmide recombinant $Ad_2D$-pBR322 de l'adénovirus, qui est constitué du fragment D $Ad_2$-BamHI inséré dans le site BamHI du pBR322, lié à un support solide, et du fragment C $Ad_2$-BamHI de l'adénovirus marqué à l'aide d'un marqueur.

11. Une combinaison de réactifs selon l'une quelconque des revendications 1 à 10, caractérisée en ce que l'un des couples de réactifs acides nucléiques est complémentaire d'une séquence d'acide nucléique à simple brin provenant d'un virus à ARN et capable de s'hybrider avec ladite séquence.

12. Une combinaison de réactifs selon l'une quelconque des revendications 1 à 11, caractérisée en ce que l'un des couples de réactifs acides nucléiques est complémentaire d'une séquence d'acide nucléique à simple brin provenant d'un virus SV40 et capable de s'hybrider avec ladite séquence.

13. Une combinaison de réactifs selon la revendication 12, caractérisée en ce que ledit couple de réactifs acides nucléiques est constitué du fragment PstI B du virus SV40 lié à un support solide et du fragment PstI A du virus SV40 marqué à l'aide d'un marqueur.

14. Une combinaison de réactifs selon l'une quelconque des revendications 1 à 13, caractérisée en ce que l'un des couples de réactifs acides nucléiques est complémentaire d'une séquence d'acide nucléique à simple brin provenant du *Bacillus amyloliquefaciens* et capable de s'hybrider avec ladite séquence.

15. Une combinaison de réactifs selon la revendication 14, caractérisée en ce que ledit couple de réactifs acides nucléiques est constitué du fragment EcoRI-BamHI du gène amylase du plasmide pKTH10 du *Bacillus amyloliquefaciens* lié à un support solide et du fragment ClaI-EcoRI du gène-amylase du plasmide pKTH10 du *Bacillus amyloliquefaciens* marqué à l'aide d'un marqueur.

16. Une combinaison de réactifs selon l'une quelconque des revendications 1 à 15, caractérisée en ce que l'un des couples de réactifs acides nucléiques est complémentaire d'une séquence d'acide nucléique à simple brin provenant d'*E. Coli* entérotoxigène et capable de s'hybrider avec ladite séquence.

17. Un kit à utiliser dans la détection de séquences d'acides nucléiques à simple brin, provenant de microorganismes par un test d'hybridation en sandwich en une étape, le kit comprenant, en combinaison conditionnée dans un coffret, au moins un couple de réactifs acides nucléiques, le ou chaque couple de réactifs étant composé de:

(a) un premier réactif acide nucléique comprenant un fragment d'acide nucléique à simple brin, lié à un support solide, et capable de former une molécule hybride à double brin par appariment des bases avec une portion d'une séquence d'acide nucléique qui peut avoir besoin d'être identifiée au moyen du kit, et

(b) un second réactif acide nucléique comprenant un fragment d'acide nucléique à simple brin, marqué à l'aide d'une marque détectable, et capable de former une molécule hybride à double brin par appariment des bases complémentaires avec une portion différente de la même séquence d'acides nucléiques en tant que paires de base avec le premier réactif acide nucléique du même couple, mais de manière que le second réactif acide nucléique ne s'hybride pas directement avec le premier réactif acide nucléique d'aucun couple.

18. Un kit selon la revendication 17, caractérisé en ce que le support solide est la nitrocellulose.

19. Un kit selon la revendication 17 ou 18, caractérisé en ce que la marque est choisie parmi les radioisotopes.

20. L'utilisation de la technique d'hybridation en sandwich de l'acide nucléique pour l'identification d'un microorganisme ou d'un groupe de microorganismes, présents dans un échantillon, ladite technique comprenant la mise en contact d'un acide nucléique à simple brin provenant d'un microorganisme ou d'un groupe de microorganismes à identifier avec un couple de réactifs acides nucléiques différents, les deux réactifs du couple étant à simple brin et complémentaires de l'acide nucléique dérivé du microorganisme, mais sensiblement incapables de s'hybrider directement l'un avec l'autre, et l'un du couple étant un fragment d'un acide nucléique lié à un support solide tandis que l'autre est un fragment d'acide nucléique marqué à l'aide d'un marqueur, ce par quoi un hybride marqué est formé, lié au support solide, le couple de réactifs acides nucléiques étant choisi en fonction du microorganisme ou groupe de microorganismes susceptibles d'être présents dans l'échantillon, et l'exactitude de l'identification étant testée en détectant le taux de formation d'un hybride marqué lié au support solide.

18

21. L'utilisation de la technique d'hybridation en sandwich des acides nucléiques selon la revendication 20, caractérisée en ce que l'on utilise une pluralité de couples de réactifs acides nucléiques, chaque couple étant complémentaire de l'acide nucléique dérivé d'un microorganisme ou d'un groupe de microorganismes différents, l'un quelconque d'entre eux étant susceptible d'être présent dans l'échantillon.

22. L'utilisation de la technique d'hybridation en sandwich des acides nucléiques selon la revendication 20, caractérisée en ce qu'une pluralité de microorganismes ou de groupes de microorganismes différents sont présents dans l'échantillon et sont détectés dans l'une et le même échantillon non divisé, par l'utilisation d'une variété de différentes couples de réactifs acides nucléiques, complémentaires des acides nucléiques provenant des microorganismes ou groupes de microorganismes différents, susceptibles d'être présents dans l'échantillon.

23. L'utilisation de la technique d'hybridation en sandwich des acides nucléiques selon l'une quelconque des revendications 20 à 22, caractérisée en ce que l'on ajoute les deux réactifs du ou de chaque couple de réactifs acides nucléiques à l'échantillon pour une hybridation par une procédure en une étape, et lorsque l'hybridation s'est produite, on mesure le taux de marquage du support ou des supports solide(s) dû à la formation d'un hybride marqué.

24. Une méthode d'identification des microorganismes ou des groupes de microorganismes présents dans un échantillon au moyen de l'hybridation en sandwich des acides nucléiques, en utilisant un couple de réactifs acides nucléiques différents, tous deux complémentaires d'un acide nucléique provenant du même microorganisme ou des mêmes groupes de microorganismes connu(s), et capables de s'hybrider avec ledit acide nucléique, l'un du couple de réactifs acides nucléiques étant un fragment d'acide nucléique à simple brin lié à un support solide, et l'autre étant un fragment d'acide nucléique à simple brin marqué à l'aide d'un marqueur, ce par quoi lors d'une hybridation réussie, un hybride marqué, lie au support solide est formé, la méthode étant caractérisée en ce que l'on identifie une pluralité de microorganismes ou de groupes de microorganismes présents dans un échantillon unique non divisé, en amenant les acides nucléiques provenant des microorganismes dans l'échantillon, après les avoir rendus à simple brin, en contact avec une pluralité de couples de réactifs acides nucléiques complètement différents, les reactifs dans les différents couples étant complémentaires de l'acide nucléique provenant de microorganismes ou de groupes de microorganismes différents et capables de s'hybrider avec ledit acide nucléique, et détectant la formation ou l'absence de formation d'hybrides marqués, liés au support solide pour chacun des couples de réactifs acides nucléiques.

25. Une méthode selon la revendication 24, caractérisée en ce que l'on ajoute simultanément à l'échantillon les deux composants acides nucléiques de chaque couple.

26. Une méthode de vérification de l'identité de l'acide nucléique à simple brin provenant d'un microorganisme au moyen de l'hybridation en sandwich de la séquence d'acide nucléique à l'aide d'un premier réactif acide nucléique qui est un fragment d'acide nucléique à simple brin lié à un support solide, et d'un second réactif acide nucléique qui est un fragment d'acide nucléique à simple brin marqué à l'aide d'un marqueur, les deux réactifs acides nucléiques étant complémentaires d'une séquence d'acides nucléiques à identifier et interagissant avec ladite séquence d'acides nucléiques si elle à l'identité attendue, pour former un hybride marqué lié au support solide, caractérisée en ce que l'on effectue l'hybridation en sandwich sous forme d'hybridation en sandwich en une étape, la séquence d'acides nucléiques à simple brin étant mise en contact en une étape avec les premier et second réactifs acides nucléiques, qui ont été choisis selon l'identité attendue de la séquence d'acides nucléiques de manière qu'ils ne s'hybrident pas directement de manière importante l'un l'autre, et l'on teste l'exactitude de l'identification de la séquence d'acides nucléiques en détectant la formation de l'hybride marqué lié au support solide.

27. Une méthode selon la revendication 26, caractérisée en ce que l'on emploie simultanément une pluralité de différents couples de premier et second réactifs acides nucléiques dans l'hybridation en sandwich en une étape, chaque couple de réactifs étant choisi en fonction d'une identité différente attendue de la séquence d'acides nucléiques.

28. Une méthode selon la revendication 26, caractérisée en ce qu'une pluralité de séquences d'acides nucléiques à simple brin est présente et en ce que l'on emploie simultanément une pluralité de différents couples de premier et second réactifs acides nucléiques dans l'hybridation en sandwich en une étape, les différents couples de réactifs acides nucléiques étant choisis en fonction d'une identité attendue de différentes séquences d'acides nucléiques.

29. Une méthode selon l'une quelconque des revendications 25 à 28, caractérisée en ce que le ou chaque acide nucléique à identifier est intrinsèquement à double brin et est dénaturé en forme à simple brin avant l'hybridation en sandwich en une étape, le matériau dénaturé étant mis en contact avec les couples de réactifs acides nucléiques de manière que la formation d'un hybride marqué lié au support solide soit en compétition avec la reconstitution de la séquence d'acide nucléique à double brin par réversion de la dénaturation.

30. Une méthode selon l'une quelconque des revendications 25 à 29, caractérisée en ce que le ou chaque premier réactif acide nucléique est un fragment d'acide nucléique sous forme de, ou dérivé d'un plasmide recombinant lié à un support solide, connu pour son emploi en tant que support de plasmide recombinant ou pour des fragments d'acides nucléiques provenant de plasmides recombinants.

31. Une méthode selon l'une quelconque des revendications 25 à 30, caractérisée en ce que le support

**0 079 139**

solide pour le premier réactif acide nucléique ou l'un ou plusieurs des premiers réactifs acides nucléiques est la nitrocellulose.

32. Une méthode selon l'une quelconque des revendications 25 à 31, caractérisée en ce que le ou chaque second réactif acide nucléique est un fragment d'acide nucléique sous forme de ou dérivé d'un plasmide recombinant marqué à l'aide d'un marqueur détectable connu pour son emploi dans le marquage des plasmides recombinants.

33. Une méthode selon l'une quelconque des revendications 25 à 29 ou 31, caractérisée en ce que le second réactif acide nucléique ou l'un ou plusieurs des seconds réactifs acides nucléiques est marqué de manière radioactive.